# EUROPEAN PATENT SPECIFICATION

(11) **EP 3 169 802 B1**
(45) Date of publication and mention of the grant of the patent: **22.05.2019**
(21) Application number: 15744821.8
(22) Date of filing: 16.07.2015
(51) Int. Cl.: C12Q 1/689

(54) **SEQUENCES AND THEIR USE FOR DETECTION OF LISTERIA MONOCYTOGENES**
SEQUENZEN UND IHRE VERWENDUNG ZUM NACHWEIS VON LISTERIA MONOCYTOGENES
SÉQUENCES ET LEUR UTILISATION POUR LA DÉTECTION DE LISTERIA MONOCYTOGENES

(30) Priority: 18.07.2014 US 201462026135 P
(43) Date of publication of application: 24.05.2017
(73) Proprietor: Qualicon Diagnostics LLC, Wilmington, DE 19803 (US)
(72) Inventor: BLUMERMAN, Seth, L., Newark, DE 19711 (US); VARKEY, Stephen, Hockessin, DE 19707 (US); JENSEN, Mark A., West Chester, PA 19382 (US); DEMARCO, Daniel R., Wilmington, DE 19806 (US)
(74) Representative: Dehns
(86) International application number: PCT/US2015/040753
(87) International publication number: WO 2016/011255

(56) References cited:
- WO-A1-98/20160
- WO-A1-2004/092406
- WO-A2-00/66777
- KOO K ET AL: "Detection of Listeria monocytogenes from a model food by fluorescence resonance energy transfer-based PCR with an asymmetric fluorogenic probe set", APPLIED AND ENVIRONMENTAL MICROBIOLOGY, AMERICAN SOCIETY FOR MICROBIOLOGY, US, vol. 69, no. 2, 1 February 2003 (2003-02-01), pages 1082-1088, XP002390691, ISSN: 0099-2240, DOI: 10.1128/AEM.69.2.1082-1088.2003

### SEQUENCE LISTING

<110> E.I. Du Pont De Nemours and Company
   Blumerman, Seth
   Varkey, Stephen
   Jensen, Mark A
<120> SEQUENCES AND THEIR USE FOR DETECTION OF LISTERIA MONOCYTOGENES
<130> MD6693WOPCT
<150> 62/026,135
   <151> 2014-07-18
<160> 17
<170> PatentIn version 3.5
<210> 1
   <211> 855
   <212> DNA
   <213> Listeria monocytogenes
<400> 1
   atgacaaatg caaatggcaa tctaaaaaaa tgccccatca cgattagctc ttacacacta        60
   ggaacggagg tatcttttcc taaacgagtg aaagtcgctg cggaaaatgg ttttgacgga       120
   attggcttgc gtgcagaaaa ttatgtagat gcactagctg ccggattaac cgatgaagac       180
   atgttgcgga ttttagacga gcataacatg aaagtaacag aagtggagta cataacccag       240
   tggggaactg ccgaagatcg tacagcagaa caacaaaaga aagagcaaac aactttccac       300
   atggcgcgat tatttggcgt caaacatatt aattgtggtt tgcttgaaaa aatccctgag       360
   gaacaaatca ttgtcgcgct tggtgaatta tgtgaccgcg cagaagaatt aattattggt       420
   ttagaattta tgccatatag cggtgtagca gacttacaag cagcttggcg agtagcagaa       480
   gcatgcggac gagataacgc gcaacttatt tgtgacacat ggcactgggc tagagcaaat       540
   caaacagctg aatctatcaa aaatgttccc gctgatcgga ttgtttctat ccaactatgc       600
   gatgtccacg aaacacctta caaagaactt cgtgaagaat cacttcatga tcgtttagct       660
   cctggagaag gatacggaga tacggtcggt tttgcaaaaa ttttaaaaga gcatggtgta       720
   aatccacgtg tgatgggagt tgaagttatt tcagactcta tggtagcaac tggtttagag       780
   tatgccgctc ttaaagtata caatgccacg aaaaaagtat tagacgaagc atggccagag       840
   atttctccac gctaa                                                        855
<210> 2
   <211> 30
   <212> DNA
   <213> Artificial Sequence
<220>
   <223> Primer
<400> 2
   acggtagaat aggttaactg tccagttcca 30
<210> 3
   <211> 30
   <212> DNA
   <213> Artificial Sequence
<220>
   <223> Primer
<400> 3
   agaataggtt aactgtccag ttccattttc 30
<210> 4
   <211> 30
   <212> DNA
   <213> Artificial Sequence
<220>
   <223> Primer
<400> 4
   ggttaactgt ccagttccat tttcaactat 30
<210> 5
   <211> 27
   <212> DNA
   <213> Artificial Sequence
<220>
   <223> Primer
<400> 5
   tttgttgttc tgctgtacga tcttcgg 27
<210> 6
   <211> 27
   <212> DNA
   <213> Artificial Sequence
<220>
   <223> Primer
<400> 6
   atgtagatgc actagctgcc ggattaa 27
<210> 7
   <211> 29
   <212> DNA
   <213> Artificial Sequence
<220>
   <223> Primer
<400> 7
   cttttgttgt tctgctgtac gatcttcgg 29
<210> 8
   <211> 27
   <212> DNA
   <213> Artificial Sequence
<220>
   <223> Primer
<400> 8
   ggtagaatag gttaactgtc cagttcc 27
<210> 9
   <211> 30
   <212> DNA
   <213> Artificial Sequence
<220>
   <223> Primer
<400> 9
   cgagataatg cgcaacttat ttgtgacacg 30
<210> 10
   <211> 27
   <212> DNA
   <213> Artificial Sequence
<220>
   <223> Primer
<400> 10
   gtaaggcgtt tcgtggacat cgcatag 27
<210> 11
   <211> 73
   <212> DNA
   <213> Artificial Sequence
<220>
   <223> Scorpion
<220>
   <221> misc_feature
   <222> (1)..(1)
   <223> 5' fluorophore (Cal Fluor Red 610)
<220>
   <221> misc_feature
   <222> (46)..(46)
   <223> Internal quencher molecule (BHQ2)
<220>
   <221> misc_feature
   <222> (46)..(47)
   <223> Internal SP-18 spacer
<400> 11
   cgtcggaaca gaagtggagt acataactca gtggggaact ccgacgtttg ttgttctgct 60
   gtacgatctt cgg 73
<210> 12
   <211> 77
   <212> DNA
   <213> Artificial Sequence
<220>
   <223> Scorpion
<220>
   <221> misc_feature
   <222> (1)..(1)
   <223> 5' fluorophore (Cal Fluor Red 610)
<220>
   <221> misc_feature
   <222> (50)..(50)
   <223> Internal quencher molecule (BHQ2)
<220>
   <221> misc_feature
   <222> (50)..(51)
   <223> Internal SP-18 spacer
<400> 12
   ccgtcgtgaa aacctctgat gtgataacgc cttcaatagt tgcacgacgg ggtagaatag 60
   gttaactgtc cagttcc 77
<210> 13
   <211> 73
   <212> DNA
   <213> Artificial Sequence
<220>
   <223> Scorpion
<220>
   <221> misc_feature
   <222> (1)..(1)
   <223> 5' fluorophore (Cal Fluor Red 610)
<220>
   <221> misc_feature
   <222> (46)..(46)
   <223> Internal quencher molecule (BHQ2)
<220>
   <221> misc_feature
   <222> (46)..(47)
   <223> Internal SP-18 spacer
<400> 13
   ggaccacggc tcgtctaaaa tccgcaacat gtcttcatcg tggtccatgt agatgcacta 60
   gctgccggat taa 73
<210> 14
   <211> 72
   <212> DNA
   <213> Artificial Sequence
<220>
   <223> Scorpion
<220>
   <221> misc_feature
   <222> (1)..(1)
   <223> 5' fluorphore (Cal Fluor Red 610)
<220>
   <221> misc_feature
   <222> (42)..(42)
   <223> Internal quencer molecule (BHQ2)
<220>
   <221> misc_feature
   <222> (42)..(43)
   <223> Internal SP-18 spacer
<400> 14
   ggccacattc tgctgtttgg tttgctctag cccagtgtgg cccgagataa tgcgcaactt        60
   atttgtgaca cg                                                            72
<210> 15
   <211> 75
   <212> DNA
   <213> Artificial Sequence
<220>
   <223> Scorpion
<220>
   <221> misc_feature
   <222> (1)..(1)
   <223> 5' fluorophore (Cal Fluor Red 610)
<220>
   <221> misc_feature
   <222> (48)..(48)
   <223> Internal quencher molecule (BHQ2)
<220>
   <221> misc_feature
   <222> (48)..(49)
   <223> Internal SP-18 spacer
<400> 15
   ccgtcgtaaa acctctgatg tgataacgcc ttcaatagtt gacgacgggg tagaataggt        60
   taactgtcca gttcc                                                         75
<210> 16
   <211> 75
   <212> DNA
   <213> Artificial Sequence
<220>
   <223> Scorpion
<220>
   <221> misc_feature
   <222> (1)..(1)
   <223> 5' fluorophore (Cal Fluor Red 610)
<220>
   <221> misc_feature
   <222> (48)..(48)
   <223> Internal quencher molecule (BHQ2)
<220>
   <221> misc_feature
   <222> (48)..(49)
   <223> Internal SP-18 spacer
<400> 16
   ccgtgtgaaa acctctgatg tgataacgcc ttcaatagtt gcacacgggg tagaataggt        60
   taactgtcca gttcc                                                          75
<210> 17
   <211> 145
   <212> DNA
   <213> Simian virus 40
<400> 17
   aatagcagac actctatgcc tgtgtggagt aagaaaaaac agtatgttat gattataact        60
   gttatgccta cttataaagg ttacagaata tttttccata attttcttgt atagcagtgc       120
   agctttttcc tttgtggtgt aaata                                              145

## Description

### CROSS REFERENCE TO RELATED APPLICATIONS

This application claims the priority of US Provisional Application Serial Number 62/026,135 filed on July 18, 2014

### FIELD OF INVENTION

The field of invention relates to methods for detection of *Listeria monocytogenes* serotypes based on the presence of nucleic acid sequences, preferably PCR-based methods for detection, and to oligonucleotide molecules and reagents and kits useful therefor.

### BACKGROUND OF INVENTION

*Listeria monocytogenes* is a causal agent of food-borne disease outbreaks associated with ready-to-eat foods such as cheeses and deli meats as well as processed foods such as hot dogs. *Listeria monocytogenes* has also been responsible for food-borne outbreaks linked to cantaloupes and other food items not typically associated with *Listeria monocytogenes* contamination. Listeriosis in the elderly and young can be very deadly with mortality rates of up to 40%. Pregnant women are also susceptible with the resulting infection causing miscarriage, stillbirth or disease of the fetus. *Listeria monocytogenes* and other species of *Listeria* are common environmental bacteria found in a variety of locales favoring their persistence. Various food production facilities may provide favorable environments which can permit the replication of *Listeria* species -including *L. monocytogenes*-and the possible contamination of raw food ingredients or finished food products. Many species of the genus *Listeria* are able to survive and continue to grow and increase in number under refrigeration temperatures. Multiple species of *Listeria* may be present in the same environment. Classical microbiologic methods for detection of *Listeria* species in food products or food preparation environments can require up to five days after the start of the screening assay. While awaiting the screening assay result, food products must be stored at considerable expense to the producer or seller of the product. Food produced in environments which subsequently test positive for the presence of *Listeria* may need to be assayed for the presence of *Listeria monocytogenes* prior to shipment or the inventory scrapped at a loss.

Therefore, there is a need for assays that detect *Listeria monocytogenes* with fast time-to results, high accuracy, and superior ease of use. Whilst WO00/66777 provides a PCR assay for detecting Listeria monocytogenes, it does not teach the use of Scorpion probes.

### SUMMARY OF INVENTION

The scope of the present invention is defined by the appended claims. Disclosed is a method for detecting the presence of *Listeria monocytogenes* in a sample, said sample comprising nucleic acids, said method comprising: (a) providing a reaction mixture comprising suitable primer pairs for amplification of at least a portion of the genes (for example, *loII*) encoding inosose isomerase in *Listeria* monocytogenes, (b) performing PCR amplification of said nucleic acids of said sample using the reaction mixture of step (a); and (c) detecting the amplification of step (b), whereby a positive detection of amplification indicates the presence of *Listeria monocytogenes* in the sample.

Also disclosed is a primer comprising a polynucleotide sequence having at least 90% sequence identity based on the BLASTN method of alignment to the polynucleotide sequence set forth in SEQ ID NOs: 2, 3, 4, 5, 6, 7, 8, 9, and 10.

Also disclosed is a primer comprising a polynucleotide sequence having at least 95% sequence identity based on the BLASTN method of alignment to the polynucleotide sequence set forth in SEQ ID NOs: 2, 3, 4, 5, 6, 7, 8, 9, and 10.

Further disclosed is a Scorpion probe comprising polynucleotide sequences having at least 90% sequence identity based on the BLASTN method of alignment to the polynucleotide sequences set forth in SEQ ID NOs: 11, 12, 13, 14, 15, and 16.

Disclosed is a Scorpion probe comprising polynucleotide sequences having at least 95% sequence identity based on the BLASTN method of alignment to the polynucleotide sequences set forth in SEQ ID NOs: 11, 12, 13, 14, 15, and 16.

The disclosure further includes a *Listeria* monocytogenes detection sequence comprising a polynucleotide sequence having at least 90% sequence identity based on the BLASTN method of alignment to the polynucleotide sequence set forth in SEQ ID NOs: 11, 12, 13, 14, 15, and 16.

Also disclosed is a genus *Listeria monocytogenes* detection sequence comprising a polynucleotide sequence having at least 95% sequence identity based on the BLASTN method of alignment to the polynucleotide sequence set forth in SEQ ID NOs: 11, 12, 13, 14, 15, and 16.

The disclosure also includes a replication composition for use in performance of PCR, comprising: (a) a set of primer pairs selected from the group consisting of: SEQ ID NOs 2, 7, and 11; SEQ ID NOs 2 and 7; SEQ ID NOs 2 and 11; SEQ ID NOs 3, 5, and 11; SEQ ID NOs 3 and 5; SEQ ID NOs 3 and 11; SEQ ID NOs 4, 5, and 11; SEQ ID NOs 4 and 5; SEQ ID NOs 4 and 11; SEQ ID NOs 5, 6, and 11; SEQ ID NOs 5 and 6; SEQ ID NOs 6 and 11; SEQ ID NOs 5, 8, and 11; SEQ ID NOs 5 and 8; SEQ ID NOs 8 and 11; SEQ ID NOs 5, 8, and 12; SEQ ID NOs 5 and 12; SEQ ID NOs 5, 6, and 13; SEQ ID NOs 5 and 13; SEQ ID NOs 9, 10, and 14; SEQ ID NOs 9 and 10; SEQ ID NOs 10 and 14; SEQ ID NOs 5, 8, and 15; SEQ ID NOs 5 and 15; SEQ ID NOs 5, 8, and 16; and, SEQ ID NOs 5 and 16 (any primer-probe-primer, primer-probe, or primer-primer pairing listed in Table 1 at paragraph 052 below) and (b) at least one DNA polymerase. The at least one DNA polymerase can be a thermostable DNA polymerase.

Also disclosed is a kit for detection of *Listeria monocytogenes*, inclusive of all serotypes, in a sample, comprising the aforementioned replication composition, wherein the kit comprises the one or more primer pairs selected from SEQ ID NOs: 2 and 7; SEQ ID NOs: 3 and 5; SEQ ID NOs: 4 and 5; SEQ ID NOs: 5 and 6; SEQ ID NOs: 5 and 8; and, SEQ ID NOs: 9 and 10 and the Scorpion probe SEQ ID NO: 11, 12, 13, 14, 15 or 16, as set forth in Table 1.

Other advantages will become apparent to those skilled in the art upon reference to the detailed description that follows hereinafter.

### SUMMARY OF THE SEQUENCES

The sequences conform with the sequence listing requirements of the EPO and PCT (Rules 5.2 and 49.5(a-*bis*), and Section 208 and Annex C of the Administrative Instructions).
SEQ ID NO:1 is the target gene sequence for *Listeria monocytogenes,* a putative *loII* inosose isomerase gene. The following is the sequence listing for
SEQ ID NOs: 2, 3, 4, 5, 6, 7, 8, 9, and 10 are primer sequences for detection of *Listeria monocytogenes* serotypes.
SEQ ID NOs: 11, 12, 13, 14, 15, and 16 are Scorpion probes for use in the detection of *Listeria monocytogenes* serotypes. In one embodiment, the probe is 5'-labeled with a fluorescent dye. In some embodiments the 3' termini of SEQ ID NOs: 11, 12, 13, 14, 15, and 16 are comprised of one of the primers listed above, for example SEQ ID NO:5 , a suitable linker moiety, such as a spacer consisting of 6 polyethylene glycol units and a quencher dye.

### DETAILED DESCRIPTION OF PREFERRED EMBODIMENTS

Further, when an amount, concentration, or other value or parameter is given as either a range, preferred range, or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

### Definitions

In this disclosure, a number of terms and abbreviations are used. The following definitions are provided.

As used herein, the term "about" or "approximately" means within 20%, preferably within 10%, and more preferably within 5% of a given value or range.

The term "comprising" is intended to include embodiments encompassed by the terms "consisting essentially of" and "consisting of". Similarly, the term "consisting essentially of" is intended to include embodiments encompassed by the term "consisting of".

The term *"Listeria monocytogenes loII* gene" or "*loII*" refers to the region(s) of the genome of *Listeria monocytogenes* serotypes that encode members of the family of inosose isomerase proteins. For reference, the gene sequence (*loII*) for the inosose isomerase is used as an example herein but other genes in the family of inosose isomerase proteins might be similarly considered. The nucleotide sequence accessioned as GenBank: HG421741.1 (range 2326424 to 2327278) is an example of the highly conserved gene sequence for various species of *Listeria monocytogenes* inosose isomerase proteins.

"Polymerase chain reaction" is abbreviated PCR.

The term "isolated" refers to materials, such as nucleic acid molecules and/or proteins, which are substantially free or otherwise removed from components that normally accompany or interact with the materials in a naturally occurring environment. Isolated polynucleotides may be purified from a host cell in which they naturally occur. Conventional nucleic acid purification methods known to skilled artisans may be used to obtain isolated polynucleotides. The term also embraces recombinant polynucleotides and chemically synthesized polynucleotides.

The terms "nucleotide", "oligonucleotide", "polynucleotide", "polynucleotide sequence", "nucleotide sequence", "nucleic acid sequence", and "nucleic acid fragment" are used interchangeably herein. These terms encompass nucleotide sequences and the like. A polynucleotide may be a polymer of RNA or DNA that is single- or double-stranded, that optionally contains synthetic, non-natural, or altered nucleotide bases. A polynucleotide may also consist of nucleotide sequences joined at the 3' end of one nucleotide sequence to the 5' end of another nucleotide sequence by a linker such as a 3 or 6 carbon (propandiol or hexandiol, repectively) moiety, or a linker arm of either 3 or 6 polyethylene glycol subunits (triethylene glycol or hexaethylene glycol, respectively). Any suitable linkers or spacers that are known in the art will work for this application. A polynucleotide in the form of a polymer of DNA may be comprised of one or more strands of cDNA, genomic DNA, synthetic DNA, or mixtures thereof.

The term "amplification product" refers to nucleic acid fragments produced during a primer-directed amplification reaction. Typical methods of primer-directed amplification include polymerase chain reaction (PCR), ligase chain reaction (LCR), or strand displacement amplification (SDA). If PCR methodology is selected, the replication composition may comprise the components for nucleic acid replication, for example: nucleotide triphosphates, two (or more) primers with appropriate sequences, thermostable polymerase, buffers, solutes, and proteins. These reagents and details describing procedures for their use in amplifying nucleic acids are provided in U.S. Patent Nos. 4,683,202 and 4,683,195, If LCR methodology is selected, then the nucleic acid replication compositions may comprise, for example: a thermostable ligase (*e*.*g*., *Thermus aquaticus* ligase), two sets of adjacent oligonucleotides (wherein one member of each set is complementary to each of the target strands), Tris-HCl buffer, KCl, EDTA, NAD, dithiothreitol, and salmon sperm DNA. *See, e*.*g.,* Tabor et al., Proc. Natl. Acad. Sci. U.S.A. 82:1074-78 (1985).

The term "primer" refers to an oligonucleotide (synthetic or occurring naturally) that is capable of acting as a point of initiation of nucleic acid synthesis or replication along a complementary strand when placed under conditions in which synthesis of a complementary strand is catalyzed by a polymerase. A primer can further contain a detectable label, for example a 5' end label.

The term "probe" refers to an oligonucleotide (synthetic or occurring naturally) that is complementary (though not necessarily fully complementary) to a polynucleotide of interest and forms a duplexed structure by hybridization with at least one strand of the polynucleotide of interest. A probe or primer-probe complex can further contain a detectable label.

A probe is complexed with or otherwise attached to a primer, such as where a probe is connected via its 3' terminus to a primers 5' terminus through a linker, which may be a nucleotide or non-nucleotide linker and which may be a non-amplifiable linker (blocker), such as a 3 or 6 carbon (propandiol or hexandiol, repectively) moiety, or a linker arm of either 3 or 6 polyethylene glycol subunits (triethylene glycol or hexaethylene glycol, respectively.. In such a case, this would be termed a "primer-probe complex". One example of such a primer-probe complex can be found in U.S. Patent No. 6,326,145, which are frequently referred to as "Scorpion probes" or "Scorpion primers".

As used herein, the terms "label" and "detectable label" refer to a molecule capable of detection, including, but not limited to, radioactive isotopes, fluorescers, chemiluminescers, enzymes, enzyme substrates, enzyme cofactors, enzyme inhibitors, chromophores, dyes, metal ions, metal sols, semiconductor nanocrystals, ligands (e.g., biotin, avidin, streptavidin, or haptens), and the like. A detectable label can also include a combination of a reporter and a quencher with the probe.

The term "reporter" refers to a substance or a portion thereof which is capable of exhibiting a detectable signal, which signal can be suppressed by a quencher. The detectable signal of the reporter is, e.g., fluorescence in the detectable range. The term "quencher" refers to a substance or portion thereof which is capable of suppressing, reducing, inhibiting, etc., the detectable signal produced by the reporter.

As used herein, the terms "quenching" and "fluorescence energy transfer" refer to the process whereby, when a reporter and a quencher are in close proximity, and the reporter is excited by an energy source, a substantial portion of the energy of the excited state nonradiatively transfers to the quencher where it either dissipates nonradiatively or is emitted at a different emission wavelength than that of the reporter.

Preferably, the reporter may be selected from fluorescent organic dyes modified with a suitable linking group for attachment to the oligonucleotide, such as to the terminal 3' carbon or terminal 5' carbon. The quencher may also be selected from organic dyes, which may or may not be fluorescent, depending on the embodiment of the present invention. Generally, whether the quencher is fluorescent or simply releases the transferred energy from the reporter by non-radiative decay, the absorption band of the quencher should at least substantially overlap the fluorescent emission band of the reporter to optimize the quenching. Non-fluorescent quenchers or dark quenchers typically function by absorbing energy from excited reporters, but do not release the energy radiatively.

Selection of appropriate reporter-quencher pairs for particular probes may be undertaken in accordance with known techniques. Fluorescent and dark quenchers and their relevant optical properties from which exemplary reporter-quencher pairs may be selected are listed and described, for example, in Berlman, Handbook of Fluorescence Spectra of Aromatic Molecules, 2nd ed., Academic Press, New York, 1971, the content of which is incorporated herein by reference. Examples of modifying reporters and quenchers for covalent attachment via common reactive groups that can be added to an oligonucleotide in the present invention may be found, for example, in Haugland, Handbook of Fluorescent Probes and Research Chemicals, Molecular Probes of Eugene, Oreg., 1992.

Preferred reporter-quencher pairs may be selected from xanthene dyes including fluoresceins and rhodamine dyes. Many suitable forms of these compounds are available commercially with substituents on the phenyl groups, which can be used as the site for bonding or as the bonding functionality for attachment to an oligonucleotide. Another preferred group of fluorescent compounds for use as reporters are the naphthylamines, having an amino group in the alpha or beta position. Included among such naphthylamino compounds are 1-dimethylaminonaphthyl-5 sulfonate, 1-anilino-8-naphthalene sulfonate and 2-p-touidinyl-6-naphthalene sulfonate. Other dyes include 3-phenyl-7-isocyanatocoumarin; acridines such as 9-isothiocyanatoacridine; N-(p-(2-benzoxazolyl)phenyl)maleimide; benzoxadiazoles; stilbenes; pyrenes and the like.

Most preferably, the reporters and quenchers are selected from fluorescein and rhodamine dyes. These dyes and appropriate linking methodologies for attachment to oligonucleotides are well known in the art.

Suitable examples of quenchers may be selected from 6-carboxy-tetramethyl-rhodamine, 4-(4-dimethylaminophenylazo) benzoic acid (DABYL), tetramethylrhodamine (TAMRA), BHQ-0™, BHQ-1™, BHQ-2™, and BHQ-3™, each of which are available from LGC Biosearch Technologies, Inc. of Novato, Calif., QSY-7™, QSY-9™, QSY-21™ and QSY-35™, each of which are available from Molecular Probes, Inc., and the like.

Suitable examples of reporters may be selected from dyes such as SYBR green, 5-carboxyfluorescein (5-FAM™ available from Applied Biosystems of Foster City, Calif.), 6-carboxyfluorescein (6-FAM), tetrachloro-6-carboxyfluorescein (TET), 2,7-dimethoxy-4,5-dichloro-6-carboxyfluorescein, hexachloro-6-carboxyfluorescein (HEX), 6-carboxy-2',4,7,7'-tetrachlorofluorescein (6-TET™ available from Applied Biosystems), carboxy-X-rhodamine (ROX), 6-carboxy-4',5'-dichloro-2',7'-dimethoxyfluorescein (6-JOE™ available from Applied Biosystems), VIC™ dye products available from Molecular Probes, Inc., NED™ dye products available from available from Applied Biosystems, Cal Fluor® dye products (such as, e.g., Cal Fluor® Gold 540, Orange 560, Red 590, Red 610, Red 635) available from LGC Biosearch Technologies, Quasar dye products (such as, e.g., Quasar 570, 670, 705) available from LGC Biosearch Technologies, and the like.

One example of a probe which contains a reporter and a quencher is a Scorpion probe in either a unimolecular or bimolecular conformation. In a unimolecular Scorpion, the probe portion of the primer-probe complex is flanked by self-complementary regions which allow the probe to form into a stem-loop structure when the probe is unbound from its target DNA. Further, in a unimolecular Scorpion, a reporter is typically attached at or near one of the self-complementary regions, such as at the 5' terminus of the Scorpion probe, and a quencher is attached at or near the other self-complementary region, such as at the 3' end of the complementary sequence and adjacent to the non-amplifiable linker, such that the quencher is in sufficiently close proximity to the reporter to cause quenching when the probe is in its stem-loop conformation. In a bimolecular Scorpion, self-complementary flanking regions are not typically employed, but rather a separate "blocking oligonucleotide" is employed in conjunction with the Scorpion probe. This blocking oligonucleotide is capable of hybridizing to the probe region of the Scorpion probe when the probe is unbound from its target DNA. Further, in a bimolecular Scorpion, the reporter is typically attached to the probe region of the Scorpion probe, such as at the 5' terminus of the Scorpion probe, while the quencher is attached to the blocking oligonucleotide, such as at the 3' terminus of the blocking oligonucleotide, such that the quencher is in sufficiently close proximity to the reporter to cause quenching when the probe is unbound from its target DNA and is instead hybridized to the blocking oligonucleotide.

Another example of a probe which contains a reporter and a quencher is a probe that is to be used in a 5'-exonuclease assay, such as the Taqman® real-time PCR technique. In this context, the oligonucleotide probe will have a sufficient number of phosphodiester linkages adjacent to its 5' end so that the 5' to 3' nuclease activity employed can efficiently degrade the bound probe to separate the reporters and quenchers. Yet another example of a probe which contains a reporter and quencher is a Molecular Beacon type probe, which contains a probe region flanked by self-complementary regions that allow the probe to form a stem-loop structure when unbound from the probe's target sequence. Such probes typically have a reporter attached at or near one terminus and a quencher attached at or near the other terminus such that the quencher is in sufficiently close proximity to the reporter to cause quenching when the probe is in its unbound, and thus stem-loop, form.

The term "replication inhibitor moiety" refers to any atom, molecule or chemical group that is attached to the 3' terminal hydroxyl group of an oligonucleotide that will block the initiation of chain extension for replication of a nucleic acid strand. Examples include, but are not limited to: 3'-deoxynucleotides (e.g., cordycepin), dideoxynucleotides, phosphate, ligands (e.g., biotin and dinitrophenol), reporter molecules (e.g., fluorescein and rhodamine), carbon chains (e.g., propanol), a mismatched nucleotide or polynucleotide, or peptide nucleic acid units. The term "non-participatory" refers to the lack of participation of a probe or primer in a reaction for the amplification of a nucleic acid molecule. Specifically a non-participatory probe or primer is one that will not serve as a substrate for, or be extended by, a DNA or RNA polymerase. A "non-participatory probe" is inherently incapable of being chain extended by a polymerase. It may or may not have a replication inhibitor moiety.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength. Hybridization and washing conditions are well known and exemplified, for example, in Sambrook, J., Fritsch, E. F. and Maniatis, T., Molecular Cloning: A Laboratory Manual, 2nd ed., Cold Spring Harbor Laboratory: Cold Spring Harbor, NY (1989), particularly Chapter 11 and Table 11.1 therein The conditions of temperature and ionic strength determine the "stringency" of the hybridization. For preliminary screening for homologous nucleic acids, low stringency hybridization conditions, corresponding to a T*m* of 55°C, can be used, e.g., 5× SSC, 0.1 % SDS, 0.25% milk, and no formamide; or 30% formamide, 5× SSC, 0.5% SDS. Moderate stringency hybridization conditions correspond to a higher T*m*, e.g., 40% formamide, with 5× or 6× SSC. Hybridization requires that the two nucleic acids contain complementary sequences, although, depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of T*m* for hybrids of nucleic acids having those sequences. The relative stability (corresponding to higher T*m*) of nucleic acid hybridizations decreases in the following order: RNA:RNA, DNA:RNA, DNA:DNA. For hybrids of greater than 100 nucleotides in length, equations for calculating T*m* have been derived (see Sambrook *et al*., *supra*, 9.50-9.51). For hybridizations with shorter nucleic acids, i.e., oligonucleotides, the position of mismatches becomes more important, and the length of the oligonucleotide determines its specificity (see Sambrook *et al*., *supra*, 11.7-11.8). In one preferred embodiment, the length for a hybridizable nucleic acid is at least about 10 nucleotides. More preferably a minimum length for a hybridizable nucleic acid is at least about 11 nucleotides, at least about 12 nucleotides, at least about 13 nucleotides, at least about 14 nucleotides, at least about 15 nucleotides, at least about 16 nucleotides, at least about 17 nucleotides, at least about 18 nucleotides, at least about 19 nucleotides, at least about 20 nucleotides, at least about 21 nucleotides, at least about 22 nucleotides, at least about 23 nucleotides, at least about 24 nucleotides, at least about 25 nucleotides, at least about 26 nucleotides, at least about 27 nucleotides, at least about 28 nucleotides, at least about 29 nucleotides, or, most preferably, at least 30 nucleotides. Furthermore, the skilled artisan will recognize that the temperature and wash solution salt concentration may be adjusted as necessary according to factors such as length of the probe.

Standard recombinant DNA and molecular cloning techniques used here are well known in the art and are described by, e.g., Sambrook *et al*. (*supra*); and by Ausubel, F. M. et al., Current Protocols in Molecular Biology, published by Greene Publishing Assoc. and Wiley-Interscience (1987).

### Genome Detection Regions

Applicants have solved the stated problem through a method that uses a *Listeria monocytogenes* species detection assay developed based on identification of a putative *loII* gene. In some embodiments, the assay incorporates unlabeled primers and Scorpion probes for detection of *Listeria monocytogenes* serotypes (and, in some embodiments, an internal positive control (e.g., sSV40)). In some embodiments, the assay also contains a passive reference for fluorescence signal normalization and to offset well-to-well signal variation.

The presently disclosed detection assay has an analytical sensitivity at 10⁴ cfu/mL or lower with *Listeria monocytogenes* serotypes in liquid cultures. Inclusivity testing with 53 strains of *Listeria monocytogenes* showed that all were detected at comparable cell densities (Table 4). Exclusivity studies with 61 strains of closely-related non *Listeria monocytogenes* species (Table 5) revealed that none of the tested samples cross-reacted with the *Listeria monocytogenes* detection assay. Testing using food enrichment samples revealed excellent sensitivity and specificity, and robustness to PCR inhibitors.

The present disclosure therefore relates to detection and identification of *Listeria monocytogenes* through the detection of a putative *loII* gene sequences. The present detection method finds utility in detection of *Listeria monocytogenes* species in any type of sample, for example in appropriate samples for food testing, environmental testing, or human or animal diagnostic testing. While examples of suitable methods for detecting these regions are included herein, it is to be understood that the invention is not limited to the methods described. Rather any suitable method can be employed to detect these DNA regions and subsequently *Listeria monocytogenes* in a sample.

### Oligonucleotides

### Scorpion Architecture

Scorpion primers are fluorogenic primer/probe oligonucleotide hybrids whose design is such that they emit light only when incorporated into the PCR product during amplification. These primers incorporate two distinct structures: 1) a target-specific PCR primer and 2) a target-specific DNA probe sequence

### Target-specific Primer

The target-specific PCR primer is covalently linked to the 3' end of the DNA probe sequence through a blocking moiety such as a 3 or 6 carbon (propandiol or hexandiol, repectively) moiety, or a linker arm of either 3 or 6 polyethylene glycol subunits. This moiety prevents polymerase extension into the probe element. In a typical PCR reaction, the primer sequence anneals to a complimentary target DNA sequence and, by the action of a DNA polymerase in the presence of nucleotides, a templated DNA synthesis reaction is initiated from the 3' end of the primer.

### DNA Probe Sequence

The DNA probe sequence of the Scorpion probe is complementary to target gene sequences synthesized by polymerase extension from the 3' end of the target-specific primer incorporated into the Scorpion probe.

### Fluorescence Quenching Mechanism

The fluorescence quenching can be accomplished through either a bimolecular or unimolecular mechanism. In a bimolecular Scorpion format, the quencher oligonucleotide is also complimentary to the DNA probe sequence. A fluorophore molecule is covalently linked to the 5' end of the DNA Probe sequence while a quencher moiety such as a BHQ (Black Hole Quencher) dye, is covalently linked to the 3' end of the quencher oligonucleotide. In a unimolecular mechanism the probe sequence is bracketed by two complementary sequences. A fluorophore molecule is covalently linked to the 5' end of the DNA probe sequence and the quencher is located at the 3' end of the complementary sequence and adjacent to the 3 or 6 carbon (propandiol or hexandiol, repectively) moiety, or a linker arm of either 3 or 6 polyethylene glycol subunits When the probe is not hybridized to the target gene sequences synthesized by polymerase extension, these complementary sequences will hybridize with each other. This brings the fluorophore and quencher into close proximity and results in quenching of the fluorophore.

Oligonucleotides of the instant invention are set forth in SEQ ID NOs: 2-16.

Oligonucleotides of the instant invention may be used as primers for PCR amplification. Preferred primer pairs and their corresponding targets, and probes are shown in Table 1.

**TABLE 1**

| **5' Primer** | **Probe** | **3' Primer** |
|---|---|---|
| | | |
| SEQ ID NO:2 | SEQ ID NO:11 | SEQ ID NO: 7 |
| SEQ ID NO:2 | | SEQ ID NO:7 |
| SEQ ID NO:2 | SEQ ID NO:11 | |
| SEQ ID NO:3 | SEQ ID NO:11 | SEQ ID NO:5 |
| SEQ ID NO:3 | | SEQ ID NO:5 |
| SEQ ID NO:3 | SEQ ID NO:11 | |
| SEQ ID NO:4 | SEQ ID NO:11 | SEQ ID NO:5 |
| SEQ ID NO:4 | | SEQ ID NO:5 |
| SEQ ID NO:4 | SEQ ID NO:11 | |
| SEQ ID NO:6 | SEQ ID NO:1 1 | SEQ ID NO:5 |
| SEQ ID NO:6 | | SEQ ID NO:5 |
| SEQ ID NO:6 | SEQ ID NO:1 1 | |
| SEQ ID NO:8 | SEQ ID NO:11 | SEQ ID NO:5 |
| SEQ ID NO:8 | | SEQ ID NO:5 |
| SEQ ID NO:8 | SEQ ID NO:11 | |
| SEQ ID NO:8 | SEQ ID NO:1 2 | SEQ ID NO:5 |
| | SEQ ID NO:1 2 | SEQ ID NO:5 |
| SEQ ID NO:6 | SEQ ID NO:1 3 | SEQ ID NO:5 |
| | SEQ ID NO:1 3 | SEQ ID NO:5 |
| SEQ ID NO:10 | SEQ ID NO:14 | SEQ ID NO:9 |
| SEQ ID NO:10 | | SEQ ID NO:9 |
| SEQ ID NO:10 | SEQ ID NO:14 | |
| SEQ ID NO:8 | SEQ ID NO:15 | SEQ ID NO:5 |
| | SEQ ID NO:15 | SEQ ID NO:5 |
| SEQ ID NO:8 | SEQ ID NO:16 | SEQ ID NO:5 |
| | SEQ ID NO:16 | SEQ ID NO:5 |

**TABLE 2**

| **SEQ ID NO:** | **Designation** | **Use** | **Nucleotide sequence (5' -> 3')** |
|---|---|---|---|
| SEQ ID NO: 2 | Lmono-174-30 | Primer | |
| SEQ ID NO: 3 | Lmono-179-30 | Primer | |
| SEQ ID NO: 4 | Lmono-185-30 | Primer | |
| SEQ ID NO: 5 | Lmono-357 | Primer | |
| SEQ ID NO: 6 | Lmono-471-27 | Primer | |
| SEQ ID NO: 7 | Lmοnο-rc533-29 | Primer | |
| SEQ ID NO: 8 | Lmono-rc737 | Primer | |
| SEQ ID NO: 9 | Lmono-821-30 | Primer | |
| SEQ ID NO: 10 | Lmono-rc897-27 | Primer | |
| SEQ ID NO: 11 | Unimolecular Lmono-357 UMO9 | Scorpion | |
| SEQ ID NO:12 | Unimolecular Lmono-RC737 UM10 | Scorpion | |
| SEQ ID NO:13 | Unimolecular Lmono-471 UM11A | Scorpion | |
| SEQ ID NO:14 | Unimolecular Lmono-821 UM12 | Scorpion | |
| SEQ ID NO: 15 | Unimolecular Lmono-RC737 UM13 | Scorpion | |
| SEQ ID NO:16 | Unimolecular Lmono-RC737 UM14 | Scorpion | |
| | | | |

In Table 2, above, the following Scorpion probe sequences are presented in the sequence listing as the entire sequence without modifications. SEQ ID NO: 11 (Unimolecular Lmono-357 UM09) is presented in the sequence listing as the entire sequence without modifications, this scorpion probe has prior to nucleotide number 1 in SEQ ID NO: 11 a 5' fluorophore, Cal Fluor Red 610 and following nucleotide number 46 in SEQ ID NO: 11 an internal BHQ2 quencher and an SP-18 spacer followed by the remaining 27 nucleotides. Similarily, SEQ ID NO: 12 (Unimolecular Lmono-RC737 UM10) is presented in the sequence listing as the entire sequence without modifications, this scorpion probe has prior to nucleotide number 1 in SEQ ID NO: 12 a 5' fluorophore, Cal Fluor Red 610 and following nucleotide number 50 in SEQ ID NO: 12 an internal BHQ2 quencher and an SP-18 spacer followed by the remaining 27 nucleotides. Similarily, SEQ ID NO: 13 (Unimolecular Lmono-471 UM11) is presented in the sequence listing as the entire sequence without modifications, this scorpion probe has prior to nucleotide number 1 in SEQ ID NO: 13 a 5' fluorophore, Cal Fluor Red 610 and following nucleotide number 46 in SEQ ID NO: 13 an internal BHQ2 quencher and an SP-18 spacer followed by the remaining 27 nucleotides. Similarily, SEQ ID NO: 14 (Unimolecular Lmono-821 UM12) is presented in the sequence listing as the entire sequence without modifications, this scorpion probe has prior to nucleotide number 1 in SEQ ID NO: 14 a 5' fluorophore, Cal Fluor Red 610 and following nucleotide number 42 in SEQ ID NO: 14 an internal BHQ2 quencher and an SP-18 spacer followed by the remaining 27 nucleotides. Similarily, SEQ ID NO: 15 (Unimolecular Lmono-RC737 UM13) is presented in the sequence listing as the entire sequence without modifications, this scorpion probe has prior to nucleotide number 1 in SEQ ID NO: 15 a 5' fluorophore, Cal Fluor Red 610 and following nucleotide number 48 in SEQ ID NO: 15 an internal BHQ2 quencher and an SP-18 spacer followed by the remaining 27 nucleotides. Similarily, SEQ ID NO: 16 (Unimolecular Lmono-RC737 UM14) is presented in the sequence listing as the entire sequence without modifications, this scorpion probe has prior to nucleotide number 1 in SEQ ID NO: 16 a 5' fluorophore, Cal Fluor Red 610 and following nucleotide number 48 in SEQ ID NO: 16 an internal BHQ2 quencher and an SP-18 spacer followed by the remaining 27 nucleotides.

These oligonucleotide primers may also be useful for other nucleic acid amplification methods such as the ligase chain reaction (LCR) (EP 0 320 308; Carrino et al., J. Microbiol. Methods 23:3-20 (1995)); nucleic acid sequence-based amplification (NASBA) (Carrino *et al*., 1995, *supra*); and self-sustained sequence replication (3SR) and "Q-Beta replicase amplification" (Pfeffer et al., Vet. Res. Commun. 19:375-407 (1995)).

The oligonucleotide primers of the present invention can also contain a detectable label, for example a 5' end label.

In addition, oligonucleotides of the present invention also may be used as hybridization probes. Hybridization using DNA probes has been frequently used for the detection of pathogens in food, clinical and environmental samples, and the methodologies are generally known to one skilled in the art. It is generally recognized that the degree of sensitivity and specificity of probe hybridization is lower than that achieved through the previously described amplification techniques. The nucleic acid probes of the present invention can also possess a detectable label, such as a reporter-quencher combination as are employed in Scorpion probe assays or in 5'-exonuclease detection assays, such as the Taqman® assay.

The 3' terminal nucleotide of the nucleic acid probe may be rendered incapable of extension by a nucleic acid polymerase in one embodiment of the invention. The DNA polymerase can be a thermostable DNA polymerase. Such blocking may be carried out, for example by the attachment of a replication inhibitor moiety, such as a reporter or quencher, to the terminal 3' carbon of the nucleic acid probe by a linking moiety, or by making the 3'-terminal nucleotide a dideoxynucleotide. In another embodiment of the invention, at least one DNA polymerase is used. Alternatively, the 3' end of the nucleic acid probe may be rendered impervious to the 3' to 5' extension activity of a polymerase by incorporating one or more modified internucleotide linkages onto the 3' end of the oligonucleotide. Minimally, the 3' terminal internucleotide linkage must be modified, however, additional internucleotide linkages may be modified. Internucleotide modifications which prevent elongation from the 3' end of the nucleic acid probe and/or which block the 3' to 5' exonuclease activity of the DNA polymerase during PCR may include phosphorothioate linkages, methylphosphonate linkages, boranophosphate linkages, and other similar polymerase-resistant internucleotide linkages. An alternative method to block 3' extension of the probe is to form an adduct at the 3' end of the probe using mitomycin C or other like antitumor antibiotics such as described in Basu et al., Biochemistry 32:4708-18 (1993). Thus, the precise mechanism by which the 3' end of the nucleic acid probe is protected from cleavage is not essential so long as the quencher is not cleaved from the nucleic acid probe.

A nucleic acid probe sequence can also optionally be employed with the primer sequence pairs of the present invention in an amplification based detection technique, such as in the 3'-exonuclease assay. primer/probe and primer/primer combinations of the present disclosure are selected from the group consisting of: SEQ ID NOs 2, 7, and 11; SEQ ID NOs 2 and 7; SEQ ID NOs 2 and 11; SEQ ID NOs 3, 5, and 11; SEQ ID NOs 3 and 5; SEQ ID NOs 3 and 11; SEQ ID NOs 4, 5, and 11; SEQ ID NOs 4 and 5; SEQ ID NOs 4 and 11; SEQ ID NOs 5, 6, and 11; SEQ ID NOs 5 and 6; SEQ ID NOs 6 and 11; SEQ ID NOs 5, 8, and 11; SEQ ID NOs 5 and 8; SEQ ID NOs 8 and 11; SEQ ID NOs 5, 8, and 12; SEQ ID NOs 5 and 12; SEQ ID NOs 5, 6, and 13; SEQ ID NOs 5 and 13; SEQ ID NOs 9, 10, and 14; SEQ ID NOs 9 and 10; SEQ ID NOs 10 and 14; SEQ ID NOs 5, 8, and 15; SEQ ID NOs 5 and 15; SEQ ID NOs 5, 8, and 16; and, SEQ ID NOs 5 and 16 (any primer-probe-primer, primer-probe, or primer-primer pairing listed in Table 1 at paragraph 052, above).

SEQ ID NOs: 11, 12, 13, 14, 15, and 16 of the present invention contain both primer and probe regions, and thus can be employed as a primer-probe complex in an appropriate assay, such as a Scorpion probe assay. This primer probe complexes of the instant invention contain a non-amplifiable linker that connects the 3' terminus of the probe region to the 5' terminus of the primer region. This non-amplifiable linker stops extension of a complementary strand from proceeding into the probe region of the primer-probe complex. Examples of such non-amplifiable linkages include hexethylene glycol (HEG) and, preferably, a linker arm of either 3 or 6 polyethylene glycol subunits. Primer-probe complexes of the present invention can also contain a self-complementary region that allows the primer-probe complex to form a stem-loop structure when the probe is unbound from its target DNA, which may be useful, for example, in bringing the reporter and quencher into sufficiently close proximity to one another to cause the reporter signal to be quenched. Preferably, SEQ NO:11, 12, 13,14, 15, and 16 is 5' end-labeled with a CAL Fluor Red 610 reporter and a BHQ-2 label at or near the non-amplifiable linker. Other reporter moieties and corresponding quenchers may be substituted.

### Assay Methods

Detection of the presence of *Listeria monocytogenes* may be accomplished in any suitable manner. Preferred methods are primer-directed amplification methods and nucleic acid hybridization methods. These methods may be used to detect *Listeria monocytogenes* isolates in a sample that is either a complex matrix or a purified culture, e.g., from an animal, environmental, or food source suspected of contamination.

A preferred embodiment of the instant invention comprises (1) culturing a complex sample mixture in a non-selective or selective growth media to resuscitate the target bacteria, (2) releasing total target bacterial DNA, and (3) subjecting the total DNA to an amplification protocol with a primer pair of the invention and optionally with a nucleic acid probe comprising a detectable label.

### Primer-Directed Amplification Assay Methods

A variety of primer-directed nucleic acid amplification methods are known in the art which can be employed in the present invention, including thermal cycling methods (e.g., PCR, RT-PCR, and LCR), as well as isothermal methods and strand displacement amplification (SDA). The preferred method is PCR.

### Sample Preparation:

The oligonucleotides and methods according to the instant invention may be used directly with any suitable clinical or environmental samples, without any need for sample preparation. In order to achieve higher sensitivity, and in situations where time is not a limiting factor, it is preferred that the samples be pre-treated and that pre-amplification enrichment is performed.

The minimum industry standard for the detection of food-borne bacterial pathogens is a method that will reliably detect the presence of one pathogen cell in 25 g of food matrix as described in Andrews et al., 1984, "Food Sample and Preparation of Sample Homogenate", Chapter 1 in Bacteriological Analytical Manual, 8th Edition, Revision A, U.S. Food and Drug Administration. In order to satisfy this stringent criterion, enrichment methods and media have been developed to enhance the growth of the target pathogen cell in order to facilitate its detection by biochemical, immunological or nucleic acid hybridization means. Typical enrichment procedures employ media that will enhance the growth and health of the target bacteria and also inhibit the growth of any background or non-target microorganisms present.

Selective media have been developed for a variety of bacterial pathogens and one of skill in the art will know to select a medium appropriate for the particular organism to be enriched, e.g. *Listeria monocytogenes.* A general discussion and recipes of non-selective and selective media are described in the FDA Bacteriological Analytical Manual on-line (http://www.fda.gov/Food/FoodScienceResearch/LaboratoryMethods/ucm0713 63.htm) and in hard copy. (1998) published and distributed by the Association of Analytical Chemists, Suite 400, 2200 Wilson Blvd, Arlington, VA 22201-3301.

After selective growth, a sample of the complex mixtures is removed for further analysis. This sampling procedure may be accomplished by a variety of means well known to those skilled in the art. In a preferred embodiment, 5 µl of the enrichment culture is removed and added to 200 µl of lysis solution containing protease and/or mutanolysin. The lysis solution is heated at 55° C for 30 min followed by protease inactivation at 95° C for 10 min, and cooled to 4° C as described in the BAX® System User's Guide, DuPont Nutrition and Health, Wilmington, DE.

### PCR Assay Methods:

A disclosed method for detecting the presence of *Listeria monocytogenes* in a sample comprises (a) performing PCR amplification using primer pairs and primer probe pairs selected from the group consisting of: SEQ ID NOs 2, 7, and 11; SEQ ID NOs 2 and 7; SEQ ID NOs 2 and 11; SEQ ID NOs 3, 5, and 11; SEQ ID NOs 3 and 5; SEQ ID NOs 3 and 11; SEQ ID NOs 4, 5, and 11; SEQ ID NOs 4 and 5; SEQ ID NOs 4 and 11; SEQ ID NOs 5, 6, and 11; SEQ ID NOs 5 and 6; SEQ ID NOs 6 and 11; SEQ ID NOs 5, 8, and 11; SEQ ID NOs 5 and 8; SEQ ID NOs 8 and 11; SEQ ID NOs 5, 8, and 12; SEQ ID NOs 5 and 12; SEQ ID NOs 5, 6, and 13; SEQ ID NOs 5 and 13; SEQ ID NOs 9, 10, and 14; SEQ ID NOs 9 and 10; SEQ ID NOs 10 and 14; SEQ ID NOs 5, 8, and 15; SEQ ID NOs 5 and 15; SEQ ID NOs 5, 8, and 16; and, SEQ ID NOs 5 and 16 (any primer-probe-primer, primer-probe, or primer-primer pairing listed in Table 1 at paragraph 052, above) to produce a PCR amplification result; and (b) detecting the amplification, whereby a positive detection of the amplification indicates the presence of *Listeria monocytogenes* in the sample.

In another preferred embodiment, prior to performing PCR amplification, a step of preparing the sample may be carried out. The preparing step may comprise at least one of the following processes: (1) bacterial enrichment, (2) separation of bacterial cells from the sample, (3) cell lysis, and (4) total DNA extraction.

### Amplification Conditions:

A skilled person will understand that any generally acceptable PCR conditions may be used for successfully detecting *Listeria monocytogenes* using the oligonucleotides of the instant disclosure and depending on the sample to be tested and other laboratory conditions, routine optimization for the PCR conditions may be necessary to achieve optimal sensitivity and specificity. Optimally, they achieve PCR amplification results from all of the intended specific target species while giving no PCR results for other, non-target species.

### Detection/Examination/Analysis:

Primer-directed amplification products can be analyzed using various methods. Homogenous detection refers to a preferred method for the detection of amplification products where no separation (such as by gel electrophoresis) of amplification products from template or primers is necessary. Homogeneous detection is typically accomplished by measuring the level of fluorescence of the reaction mixture during or immediately following amplification. In addition, heterogeneous detection methods, which involve separation of amplification products during or prior to detection, can be employed in the present invention.

Homogenous detection may be employed to carry out "real-time" primer-directed nucleic acid amplification and detection, using primer pairs of the instant invention (e.g., "real-time" PCR and "real-time" RT-PCR). Preferred "real-time" methods are set forth in U.S. Patent Nos. 6,171,785, 5,994,056, 6,326,145, 5,804,375, 5,538,848, 5,487,972, and 5,210,015.

A particularly preferred "real-time" detection method is the Scorpion probe assay as set forth in U.S. Patent No. 6,326,145. In the Scorpion probe assay, PCR amplification is performed using a Scorpion probe (either unimolecular or bimolecular) as a primer-probe complex, the Scorpion probe possessing an appropriate reporter-quencher pair to allow the detectable signal of the reporter to be quenched prior to elongation of the primer. Post-elongation, the quenching effect is eliminated and the amount of signal present is quantitated. As the amount of amplification product increases, an equivalent increase in detectable signal will be observed, thus allowing the amount of amplification product present to be determined as a function of the amount of detectable signal measured. When more than one Scorpion probe is employed in a Scorpion probe assay each probe can have a different detectable label (e.g., reporter-quencher pair) attached, thus allowing each probe to be detected independently of the other probes.

Another preferred "real-time" detection method is the 5'-exonuclease detection method, as set forth in U.S. Patent Nos. 5,804,375, 5,538,848, 5,487,972, and 5,210,015. In the 5'-exonuclease detection assay a modified probe is employed during PCR which binds intermediate to or between the two members of the amplification primer pair. The modified probe possesses a reporter and a quencher and is designed to generate a detectable signal to indicate that it has hybridized with the target nucleic acid sequence during PCR. As long as both the reporter and the quencher are on the probe, the quencher stops the reporter from emitting a detectable signal. However, as the polymerase extends the primer during amplification, the intrinsic 5' to 3' nuclease activity of the polymerase degrades the probe, separating the reporter from the quencher, and enabling the detectable signal to be emitted. Generally, the amount of detectable signal generated during the amplification cycle is proportional to the amount of product generated in each cycle.

It is well known that the efficiency of quenching is a strong function of the proximity of the reporter and the quencher, i.e., as the two molecules get closer, the quenching efficiency increases. As quenching is strongly dependent on the physical proximity of the reporter and quencher, the reporter and the quencher are preferably attached to the probe within a few nucleotides of one another, usually within 30 nucleotides of one another, more preferably with a separation of from about 6 to 16 nucleotides. Typically, this separation is achieved by attaching one member of a reporter-quencher pair to the 5' end of the probe and the other member to a nucleotide about 6 to 16 nucleotides away.

Again, when more than one Taqman® probe is employed in a 5'-exonuclease detection assay, each probe can have a different detectable label (e.g., reporter-quencher pair) attached, thus allowing each probe to be detected independently of the other probes.

Another preferred method of homogenous detection involves the use of DNA melting curve analysis, particularly with the BAX® System hardware and reagent tablets from DuPont Nutrition and Health. The details of the system are given in U.S. Patent No. 6,312,930 and PCT Publication Nos. WO 97/11197 and WO 00/66777.

Melting curve analysis detects and quantifies double stranded nucleic acid molecule ("dsDNA" or "target") by monitoring the fluorescence of the target amplification product ("target amplicon") during each amplification cycle at selected time points.

As is well known to the skilled artisan, the two strands of a dsDNA separate or melt, when the temperature is higher than its melting temperature. Melting of a dsDNA molecule is a process, and under a given solution condition, melting starts at a temperature (designated Tms hereinafter), and completes at another temperature (designated Tme hereinafter). The familiar term, Tm, designates the temperature at which melting is 50% complete.

A typical PCR cycle involves a denaturing phase where the target dsDNA is melted, a primer annealing phase where the temperature optimal for the primers to bind to the now-single-stranded target, and a chain elongation phase (at a temperature Te) where the temperature is optimal for DNA polymerase to function.

According to the present invention, Tms should be higher than Te, and Tme should be lower (often substantially lower) than the temperature at which the DNA polymerase is heat-inactivated. Melting characteristics are affected by the intrinsic properties of a given dsDNA molecule, such as deoxynucleotide composition and the length of the dsDNA.

Intercalating dyes will bind to double stranded DNA. The dye/dsDNA complex will fluoresce when exposed to the appropriate excitation wavelength of light, which is dye dependent, and the intensity of the fluorescence may be proportionate to concentration of the dsDNA. Methods taking advantage of the use of DNA intercalating dyes to detect and quantify dsDNA are known in the art. Many dyes are known and used in the art for these purposes. The instant methods also take advantage of such relationship.

Examples of such intercalating dyes include, but are not limited to, SYBR Green-I®, ethidium bromide, propidium iodide, TOTO®-1 {Quinolinium, 1-1'-[1,3-propanediylbis[(dimethyliminio)-3,1-propanediyl]]bis[4-[(3-methyl-2(3H)-benzothiazolylidene)methyl]]-, tetraiodide}, and YoPro® {Quinolinium, 4-[(3-methyl-2(3H)-benzoxazolylidene)methyl]-1-[3-(trimethylammonio)-propyl]-,diiodide}. Most preferred for the instant invention is a non-asymmetrical cyanide dye such as SYBR Green-I®, manufactured by Molecular Probes, Inc. (Eugene, OR).

Melting curve analysis is achieved by monitoring the change in fluorescence while the temperature is increased. When the temperature reaches the T_{MS} specific for the target amplicon, the dsDNA begins to denature. When the dsDNA denatures, the intercalating dye dissociates from the DNA and fluorescence decreases. Mathematical analysis of the negative of the change of the log of fluorescence divided by the change in temperature plotted against the temperature results in the graphical peak known as a melting curve.

It should be understood that the present invention could be operated using a combination of these techniques, such as by having a Scorpion probe directed to one target region and a Taqman® probe directed to a second target region. It should also be understood that the invention is not limited to the above described techniques. Rather, one skilled in the art would recognize that other techniques for detecting amplification as known in the art may also be used. For example, techniques such as PCR-based quantitative sequence detection (QSD) may be performed using nucleic acid probes which, when present in the single-stranded state in solution, are configured such that the reporter and quencher are sufficiently close to substantially quench the reporter's emission. However, upon hybridization of the intact reporter-quencher nucleic acid probe with the amplified target nucleic acid sequence, the reporter and quenchers become sufficiently distant from each other. As a result, the quenching is substantially abated causing an increase in the fluorescence emission detected.

In addition to homogenous detection methods, a variety of other heterogeneous detection methods are known in the art which can be employed in the present invention, including standard non-denaturing gel electrophoresis (e.g., acrylamide or agarose), denaturing gradient gel electrophoresis, and temperature gradient gel electrophoresis. Standard non-denaturing gel electrophoresis is a simple and quick method of PCR detection, but may not be suitable for all applications.

Denaturing Gradient Gel Electrophoresis (DGGE) is a separation method that detects differences in the denaturing behavior of small DNA fragments (200-700 bp). The principle of the separation is based on both fragment length and nucleotide sequence. In fragments that are the same length, a difference as little as one base pair can be detected. This is in contrast to non-denaturing gel electrophoresis, where DNA fragments are separated only by size. This limitation of non-denaturing gel electrophoresis results because the difference in charge density between DNA molecules is near neutral and plays little role in their separation. As the size of the DNA fragment increases, its velocity through the gel decreases.

DGGE is primarily used to separate DNA fragments of the same size based on their denaturing profiles and sequence. Using DGGE, two strands of a DNA molecule separate, or melt, when heat or a chemical denaturant is applied. The denaturation of a DNA duplex is influenced by two factors: 1) the hydrogen bonds formed between complimentary base pairs (since GC rich regions melt at higher denaturing conditions than regions that are AT rich); and 2) the attraction between neighboring bases of the same strand, or "stacking". Consequently, a DNA molecule may have several melting domains with each of their individual characteristic denaturing conditions determined by their nucleotide sequence. DGGE exploits the fact that otherwise identical DNA molecules having the same length and DNA sequence, with the exception of only one nucleotide within a specific denaturing domain, will denature at different temperatures or T*m*. Thus, when the double-stranded (ds) DNA fragment is electrophoresed through a gradient of increasing chemical denaturant it begins to denature and undergoes both a conformational and mobility change. The dsDNA fragment will travel faster than a denatured single-stranded (ss) DNA fragment, since the branched structure of the single-stranded moiety of the molecule becomes entangled in the gel matrix. As the denaturing environment increases, the dsDNA fragment will completely dissociate and mobility of the molecule through the gel is retarded at the denaturant concentration at which the particular low denaturing domains of the DNA strand dissociate. In practice, the electrophoresis is conducted at a constant temperature (around 60 °C) and chemical denaturants are used at concentrations that will result in 100% of the DNA molecules being denatured (i.e., 40% formamide and 7 M urea). This variable denaturing gradient is created using a gradient maker, such that the composition of each DGGE gel gradually changes from 0% denaturant up to 100% denaturant. Of course, gradients containing a reduced range of denaturant (e.g., 35% to 60%) may also be poured for increased separation of DNA.

The principle used in DGGE can also be applied to a second method that uses a temperature gradient instead of a chemical denaturant gradient. This method is known as Temperature Gradient Gel Electrophoresis (TGGE). This method makes use of a temperature gradient to induce the conformational change of dsDNA to ssDNA to separate fragments of equal size with different sequences. As in DGGE, DNA fragments with different nucleotide sequences will become immobile at different positions in the gel. Variations in primer design can be used to advantage in increasing the usefulness of DGGE for characterization and identification of the PCR products. These methods and principles of using primer design variations are described in PCR Technology Principles and Applications, Henry A. Erlich Ed., M. Stockton Press, NY, pages 71 to 88 (1988).

### Instrumentation:

When homogenous detection is employed, the level of fluorescence is preferably measured using a laser fluorometer such as, for example, BAX® System Q7 machine (DuPont Nutrition and Health, Wilmington, DE). However, similar detection systems for measuring the level of fluorescence in a sample are included in the invention.

### Reagents and Kits:

Any suitable nucleic acid replication composition ("replication composition") in any format can be used. A typical replication composition for PCR amplification may comprise, for example, dATP, dCTP, dGTP, dTTP, target specific primers and at least one suitable polymerase.

If the replication composition is in liquid form, suitable buffers known in the art may be used (Sambrook, J. *et al*., *supra*).

Alternatively, if the replication composition is contained in a tablet form, then typical tabletization reagents may be included such as stabilizers and binding agents. Preferred tabletization technology is set forth in U.S. Patent Nos. 4,762,857 and 4,678,812.

A preferred replication composition of the instant invention comprises (a) a set of primer pairs or primer-probe pairs selected from the group consisting of: SEQ ID NOs 2, 7, and 11; SEQ ID NOs 2 and 7; SEQ ID NOs 2 and 11; SEQ ID NOs 3, 5, and 11; SEQ ID NOs 3 and 5; SEQ ID NOs 3 and 11; SEQ ID NOs 4, 5, and 11; SEQ ID NOs 4 and 5; SEQ ID NOs 4 and 11; SEQ ID NOs 5, 6, and 11; SEQ ID NOs 5 and 6; SEQ ID NOs 6 and 11; SEQ ID NOs 5, 8, and 11; SEQ ID NOs 5 and 8; SEQ ID NOs 8 and 11; SEQ ID NOs 5, 8, and 12; SEQ ID NOs 5 and 12; SEQ ID NOs 5, 6, and 13; SEQ ID NOs 5 and 13; SEQ ID NOs 9, 10, and 14; SEQ ID NOs 9 and 10; SEQ ID NOs 10 and 14; SEQ ID NOs 5, 8, and 15; SEQ ID NOs 5 and 15; SEQ ID NOs 5, 8, and 16; and, SEQ ID NOs 5 and 16 (any primer-probe-primer, primer-probe, or primer-primer pairing listed in Table 1 at paragraph 052 below) and (b) thermostable DNA polymerase.

A more preferred replication composition of the present invention comprises (a) the primer pairs and any corresponding probe selected from Table 1, wherein each nucleic acid probe or primer-probe complex employed comprises a detectable label; and (b) thermostable DNA polymerase. Preferably the detectable label comprises a reporter capable of emitting a detectable signal and a quencher capable of substantially quenching the reporter and preventing the emission of the detectable signal when the reporter and quencher are in sufficiently close proximity to one another.

A preferred kit of the instant invention comprises any one of the above replication compositions. A preferred tablet of the instant invention comprises any one of the above replication compositions. More preferably, a kit of the instant invention comprises the foregoing preferred tablet.

In some instances, an internal positive control can be included in the reaction. The internal positive control can include control template nucleic acids (e.g. DNA or RNA), control primers, and control nucleic acid probe. The advantages of an internal positive control contained within a PCR reaction have been previously described (U.S. Patent No. 6,312,930 and PCT Application No. WO 97/11197) and include: (i) the control may be amplified using a single primer; (ii) the amount of the control amplification product is independent of any target DNA or RNA contained in the sample; (iii) the control DNA can be tableted with other amplification reagents for ease of use and high degree of reproducibility in both manual and automated test procedures; (iv) the control can be used with homogeneous detection, i.e., without separation of product DNA from reactants; and (v) the internal control has a melting profile that is distinct from other potential amplification products in the reaction and/or a detectable label on the control nucleic acid that is distinct from the detectable label on the nucleic acid probe directed to the target.

Control DNA will be of appropriate size and base composition to permit amplification in a primer-directed amplification reaction. The control template DNA sequence may be obtained from the genome of *Listeria monocytogenes* or from another source, but must be reproducibly amplified under the same conditions that permit the amplification of the target amplification product.

Preferred control sequences include, for example, those found in SV40. The preferred concentration range of SV40, when used, is 10¹ to 10⁷ copies per PCR reaction.

The control reaction is useful to validate the amplification reaction. Amplification of the control DNA occurs within the same reaction tube as the sample that is being tested, and therefore indicates a successful amplification reaction when samples are target negative, i.e. no target amplification product is produced. In order to achieve significant validation of the amplification reaction, a suitable number of copies of the control DNA template must be included in each amplification reaction.

In some instances it may be useful to include an additional negative control replication composition. The negative control replication composition will contain the same reagents as the replication composition but without the polymerase. The primary function of such a control is to monitor spurious background fluorescence in a homogeneous format when the method employs a fluorescent means of detection.

Replication compositions may be modified depending on whether they are designed to be used to amplify target DNA or the control DNA. Replication compositions that will amplify the target DNA (test replication compositions) may include (i) a polymerase (generally thermostable), (ii) a primer pair capable of hybridizing to the target DNA and (iii) necessary buffers for the amplification reaction to proceed. Replication compositions that will amplify the control DNA (positive control, or positive replication composition) may include (i) a polymerase (generally thermostable) (ii) the control DNA; (iii) at least one primer capable of hybridizing to the control DNA; and (iv) necessary buffers for the amplification reaction to proceed. In addition, the replication composition for either target DNA or control DNA amplification can contain a nucleic acid probe, preferably possessing a detectable label.

### Nucleic Acid Hybridization Methods

In addition to primer-directed amplification assay methods, nucleic acid hybridization assay methods can be employed in the present invention for detection of *Listeria monocytogenes.* The basic components of a nucleic acid hybridization test include probe(s), a sample suspected of containing *Listeria monocytogenes,* and a specific hybridization method. Typically the probe(s) length can vary from as few as five bases to the full length of the *Listeria monocytogenes* diagnostic sequence and will depend upon the specific test to be done. Only part of the probe molecule need be complementary to the nucleic acid sequence to be detected. In addition, the complementarity between the probe(s) and the target sequence(s) need not be perfect. Hybridization does occur between imperfectly complementary molecules with the result that a certain fraction of the bases in the hybridized region(s) are not paired with the proper complementary base.

A probe particularly useful in nucleic acid hybridization methods is any of SEQ ID NOs: 2-16 or sequences derived therefrom.

The sample may or may not contain *Listeria monocytogenes.* The sample may take a variety of forms, however will generally be extracted from an animal, environmental or food source suspected of contamination. The DNA may be detected directly but most preferably, the sample nucleic acid must be made available to contact the probe before any hybridization of probe(s) and target molecule(s) can occur. Thus the organism's DNA is preferably free from the cell and placed under the proper conditions before hybridization can occur. Methods of in-solution hybridization necessitate the purification of the DNA in order to be able to obtain hybridization of the sample DNA with the probe(s). This has meant that utilization of the in-solution method for detection of target sequences in a sample requires that the nucleic acids of the sample must first be purified to eliminate protein, lipids, and other cell components, and then contacted with the probe(s) under hybridization conditions. Methods for the purification of the sample nucleic acid are common and well known in the art (Sambrook *et al*., *supra*).

In one preferred embodiment, hybridization assays may be conducted directly on cell lysates, without the need to extract the nucleic acids. This eliminates several steps from the sample-handling process and speeds up the assay. To perform such assays on crude cell lysates, a chaotropic agent is typically added to the cell lysates prepared as described above. The chaotropic agent stabilizes nucleic acids by inhibiting nuclease activity. Furthermore, the chaotropic agent allows sensitive and stringent hybridization of short oligonucleotide probes to DNA at room temperature (Van Ness & Chen, Nucleic Acids Res. 19:5143-51 (1991)). Suitable chaotropic agents include guanidinium chloride, guanidinium thiocyanate, sodium thiocyanate, lithium tetrachloroacetate, sodium perchlorate, rubidium tetrachloroacetate, potassium iodide, and cesium trifluoroacetate, among others. Typically, the chaotropic agent will be present at a final concentration of about 3 M. If desired, one can add formamide to the hybridization mixture, typically 30-50% (v/v).

Alternatively, one can purify the sample nucleic acids prior to probe hybridization. A variety of methods are known to one of skill in the art (e.g., phenol-chloroform extraction, IsoQuick extraction (MicroProbe Corp., Bothell, WA), and others). Pre-hybridization purification is particularly useful for standard filter hybridization assays. Furthermore, purification facilitates measures to increase the assay sensitivity by incorporating *in vitro* RNA amplification methods such as self-sustained sequence replication (see for example Fahy et al., In PCR Methods and Applications, Cold Spring Harbor Laboratory: Cold Spring Harbor, NY (1991), pp. 25-33) or reverse transcriptase PCR (Kawasaki, In PCR Protocols: A Guide to Methods and Applications, M. A. Innis et al., Eds., (1990), pp. 21-27).

Once the DNA is released, it can be detected by any of a variety of methods. However, the most useful embodiments have at least some characteristics of speed, convenience, sensitivity, and specificity.

Hybridization methods are well known in the art. Typically the probe and sample must be mixed under conditions which will permit nucleic acid hybridization. This involves contacting the probe and sample in the presence of an inorganic or organic salt under the proper concentration and temperature conditions. The probe and sample nucleic acids must be in contact for a long enough time that any possible hybridization between the probe and sample nucleic acid may occur. The concentration of probe or target in the mixture will determine the time necessary for hybridization to occur. The higher the probe or target concentration, the shorter the hybridization incubation time needed.

Various hybridization solutions can be employed. Typically, these comprise from about 20 to 60% volume, preferably 30%, of a polar organic solvent. A common hybridization solution employs about 30-50% v/v formamide, about 0.15 to 1M sodium chloride, about 0.05 to 0.1M buffers, such as sodium citrate, Tris-HCl, PIPES or HEPES (pH range about 6-9), about 0.05 to 0.2% detergent, such as sodium dodecylsulfate, or between 0.5-20 mM EDTA, FICOLL (Pharmacia Inc.) (about 300-500 kilodaltons), polyvinylpyrrolidone (about 250-500 kdal), and serum albumin. Also included in the typical hybridization solution will be unlabeled carrier nucleic acids from about 0.1 to 5 mg/mL, fragmented nucleic DNA (e.g., calf thymus or salmon sperm DNA, or yeast RNA), and optionally from about 0.5 to 2% wt/vol glycine. Other additives may also be included, such as volume exclusion agents which include a variety of polar water-soluble or swellable agents (e.g., polyethylene glycol), anionic polymers (e.g., polyacrylate or polymethylacrylate), and anionic saccharidic polymers (e.g., dextran sulfate).

Nucleic acid hybridization is adaptable to a variety of assay formats. One of the most suitable is the sandwich assay format. The sandwich assay is particularly adaptable to hybridization under non-denaturing conditions. A primary component of a sandwich-type assay is a solid support. The solid support has adsorbed to it or covalently coupled to it immobilized nucleic acid probe that is unlabeled and complementary to one portion of the DNA sequence.

The sandwich assay may be encompassed in an assay kit. This kit would include a first component for the collection of samples suspected of contamination and buffers for the disbursement and lysis of the sample. A second component would include media in either dry or liquid form for the hybridization of target and probe polynucleotides, as well as for the removal of undesirable and nonduplexed forms by washing. A third component includes a solid support (dipstick) upon which is fixed (or to which is conjugated) unlabeled nucleic acid probe(s) that is (are) complementary to one or more of the sequences disclosed herein. A fourth component would contain labeled probe that is complementary to a second and different region of the same DNA strand to which the immobilized, unlabeled nucleic acid probe of the third component is hybridized.

In a preferred embodiment, polynucleotide sequences disclosed herein or derivations thereof may be used as 3' blocked detection probes in either a homogeneous or heterogeneous assay format. For example, a probe generated from these sequences may be 3' blocked or non-participatory and will not be extended by, or participate in, a nucleic acid amplification reaction. Additionally, the probe incorporates a label that can serve as a reactive ligand that acts as a point of attachment for the immobilization of the probe/analyte hybrid or as a reporter to produce detectable signal. Accordingly, genomic or cDNA isolated from a sample suspected of *Listeria monocytogenes* contamination is amplified by standard primer-directed amplification protocols in the presence of an excess of the 3' blocked detection probe(s) to produce amplification products. Because the probe(s) is 3' blocked, it does not participate or interfere with the amplification of the target. After the final amplification cycle, the detection probe(s) anneals to the relevant portion of the amplified DNA and the annealed complex is then captured on a support through the reactive ligand.

In some instances it is desirable to incorporate a ligand labeled dNTP, with the label probe in the replication composition to facilitate immobilization of the PCR reaction product on a support and then detection of the immobilized product by means of the labeled probe reagent. For example a biotin, digoxigenin, or digoxin labeled dNTP could be added to PCR reaction composition. The biotin, digoxigenin, or digoxin incorporated in the PCR product could then be immobilized respectively on to a streptavidin, anti-digoxin or anti-digoxigenin antibody support. The immobilized PCR product could then be detected by the presence of the probe label.

### EXAMPLES

The present invention is further defined in the following Examples. It should be understood that these Examples, while indicating preferred embodiments of the invention, are given by way of illustration only.

### General Methods and Materials Used in the Examples

Materials and methods suitable for the maintenance and growth of bacterial cultures are well known in the art. Techniques suitable for use in the following Examples may be found in Manual of Methods for General Bacteriology (Phillipp Gerhardt, R. G. E. Murray, Ralph N. Costilow, Eugene W. Nester, Willis A. Wood, Noel R. Krieg and G. Briggs Phillips, eds), American Society for Microbiology, Washington, DC (1994) or Thomas D. Brock in Biotechnology: A Textbook of Industrial Microbiology, Second Edition (1989) Sinauer Associates, Inc., Sunderland, MA or Bacteriological Analytical Manual. 6th Edition, Association of Official Analytical Chemists, Arlington, VA (1984).

Primers and probes (SEQ ID NOs: 2-16) were prepared by LGC Biosearch Technologies, Inc., 2199 S. McDowell Blvd., Petaluma, CA 94954 USA.

All PCR reactions were carried out using a standard BAX® Q7 System (DuPont Nutrition and Health, Wilmington, DE).

The meaning of abbreviations is as follows: "h" means hour(s), "min" means minute(s), "sec" means second(s), "d" means day(s), "ml" means milliliter(s), "µl" means microliter(s), "cfu" means colony forming unit(s), "M" means molar, "µM" means micromolar, "nM" means nanomolar, "L. mono" means *Listeria monocytogenes*, Ct" means cycle threshold, "IPC" means internal positive control, "IoII" means inosose isomerase protein gene.

### EXAMPLE 1

### Development of Listeria monocytogenes Primers and Scorpion

A panel of nine primers and six unimolecular scorpions were evaluated, see Table 1. Analysis of PCR products by gel revealed amplification of *Listeria monocytogenes* specific target when tested at primer concentrations ranging from 80 nM to 640 nM. PCR reaction conditions were optimized to minimize the Ct when amplifying low levels of *Listeria monocytogenes* genomic DNA as target, while maintaining lower overall total primer concentrations to minimize primer dimer formation.

Amplification was evaluated with a low primer (SEQ ID NOs: 2-10) concentration, titrating the concentration of probe (SEQ ID NO:11-16), to determine the effects on signal amplitude and baseline fluorescence intensity, and to elucidate if this would have any impact on Ct values at a low DNA target level. Scorpion probe (SEQ ID NO:11-16) concentration was titrated from 5 nM to 40 nM. Based on titration results, 15 nM of SEQ ID NO: 11-16 showed the best PCR and Scorpion performance in terms of baseline fluorescence, fluorescence signal amplitude, and Ct values.

### EXAMPLE 2

### Inclusivity Assays

A total of 53 strains of *Listeria monocytogenes* strains, of varying serotypes and originally isolated from a variety of sources, from the internal DuPont Nutrition & Health culture collection (designated as "DD") were grown in Brain-Heart Infusion Broth (BHI) overnight at 35°C. Cells were diluted to approximately 10⁵ CFU/ml and lysed using the standard BAX lysis protocol, and tested by the real-time Listeria monocytogenes detection assay. All showed positive signals (Table 3). A subset of 13 isolates, representing a panel of *Listeria monocytogenes* serotypes(#2, 3, 4, 5, 10, 14, 22, 29, 33, 35 and 39 listed in Table 2, as well as DD651 and DD1423), were serially diluted to approximately 10² cfu/mL and tested by the real-time PCR assay. All strains tested positive at 10³ cfu/mL and higher.

**Table 3**

| # | DD# | | Assay result |
|---|---|---|---|
| 1 | 566 | *Listeria monocytogenes* | Positive |
| 2 | 605 | *Listeria monocytogenes* | Positive |
| 3 | 647 | *Listeria monocytogenes* | Positive |
| 4 | 648 | *Listeria monocytogenes* | Positive |
| 5 | 652 | *Listeria monocytogenes* | Positive |
| 6 | 653 | *Listeria monocytogenes* | Positive |
| 7 | 1069 | *Listeria monocytogenes* | Positive |
| 8 | 1072 | *Listeria monocytogenes* | Positive |
| 9 | 1144 | *Listeria monocytogenes* | Positive |
| 10 | 1145 | *Listeria monocytogenes* | Positive |
| 11 | 1146 | *Listeria monocytogenes* | Positive |
| 12 | 1147 | *Listeria monocytogenes* | Positive |
| 13 | 1149 | *Listeria monocytogenes* | Positive |
| 14 | 1152 | *Listeria monocytogenes* | Positive |
| 15 | 1281 | *Listeria monocytogenes* | Positive |
| 16 | 1282 | *Listeria monocytogenes* | Positive |
| 17 | 1283 | *Listeria monocytogenes* | Positive |
| 18 | 1285 | *Listeria monocytogenes* | Positive |
| 19 | 1286 | *Listeria monocytogenes* | Positive |
| 20 | 1287 | *Listeria monocytogenes* | Positive |
| 21 | 1288 | *Listeria monocytogenes* | Positive |
| 22 | 1293 | *Listeria monocytogenes* | Positive |
| 23 | 1294 | *Listeria monocytogenes* | Positive |
| 24 | 1295 | *Listeria monocytogenes* | Positive |
| 25 | 1299 | *Listeria monocytogenes* | Positive |
| 26 | 1302 | *Listeria monocytogenes* | Positive |
| 27 | 1305 | *Listeria monocytogenes* | Positive |
| 28 | 1306 | *Listeria monocytogenes* | Positive |
| 29 | 1307 | *Listeria monocytogenes* | Positive |
| 30 | 1308 | *Listeria monocytogenes* | Positive |
| 31 | 1309 | *Listeria monocytogenes* | Positive |
| 32 | 1310 | *Listeria monocytogenes* | Positive |
| 33 | 1311 | *Listeria monocytogenes* | Positive |
| 34 | 1312 | *Listeria monocytogenes* | Positive |
| 35 | 1313 | *Listeria monocytogenes* | Positive |
| 36 | 1314 | *Listeria monocytogenes* | Positive |
| 37 | 1315 | *Listeria monocytogenes* | Positive |
| 38 | 1316 | *Listeria monocytogenes* | Positive |
| 39 | 1321 | *Listeria monocytogenes* | Positive |
| 40 | 3573 | *Listeria monocytogenes* | Positive |
| 41 | 3574 | *Listeria monocytogenes* | Positive |
| 42 | 3576 | *Listeria monocytogenes* | Positive |
| 43 | 3577 | *Listeria monocytogenes* | Positive |
| 44 | 3578 | *Listeria monocytogenes* | Positive |
| 45 | 3579 | *Listeria monocytogenes* | Positive |
| 46 | 3580 | *Listeria monocytogenes* | Positive |
| 47 | 3581 | *Listeria monocytogenes* | Positive |
| 48 | 3582 | *Listeria monocytogenes* | Positive |
| 49 | 4553 | *Listeria monocytogenes* | Positive |
| 50 | 4568 | *Listeria monocytogenes* | Positive |
| 51 | 4571 | *Listeria monocytogenes* | Positive |
| 52 | 5425 | *Listeria monocytogenes* | Positive |
| 53 | 7644 | *Listeria monocytogenes* | Positive |

### EXAMPLE 3

### Exclusivity Assays

Sixty-one isolates of non-*Listeria monocytogenes* strains, including other species of the genus *Listeria,* were grown in BHI overnight at either 30°C or 37°C. Each strain was diluted 1:10 in BHI broth and cell lysates were prepared using the standard BAX lysis protocol, and tested by the real-time *Listeria monocytogenes* PCR assay. All strains tested negative. (Table 4).

**Table 4**

| # | DD# | Strain | Assay Result |
|---|---|---|---|
| 1 | 379 | *Bacillus subtilis* | Negative |
| 2 | 383 | *Citrobacter freundii* | Negative |
| 3 | 643 | *Listeria murrayi* / *grayi* | Negative |
| 4 | 649 | *Listeria ivanovii* | Negative |
| 5 | 654 | *Listeria welshimeri* | Negative |
| 6 | 659 | *Lactococcus lactis* | Negative |
| 7 | 691 | *Streptococcus thermophilus* | Negative |
| 8 | 692 | *Streptococcus bovis* | Negative |
| 9 | 695 | *Streptococcus pyogenes* | Negative |
| 10 | 707 | *Salmonella* Newport | Negative |
| 11 | 713 | *Bacillus thuringienses* | Negative |
| 12 | 714 | *Bacillus thuringienses* | Negative |
| 13 | 715 | *Bacillus cereus* | Negative |
| 14 | 716 | *Bacillus thwingienses* | Negative |
| 15 | 721 | *Bacillus cereus* | Negative |
| 16 | 863 | *Staphylococcus aureus* | Negative |
| 17 | 877 | *Bacillus cereus* | Negative |
| 18 | 878 | *Bacillus cereus* | Negative |
| 19 | 879 | *Bacillus cereus* | Negative |
| 20 | 912 | *Staphylococcus aureus* | Negative |
| 21 | 944 | *Listeria murrayi* / *grayi* | Negative |
| 22 | 1011 | *Bacillus subtilus* | Negative |
| 23 | 1024 | *Bacillus cereus* | Negative |
| 24 | 1096 | *Staphylococcus aureus* | Negative |
| 25 | 1098 | *Staphylococcus aureus* | Negative |
| 26 | 1105 | *Staphylococcus warnerii* | Negative |
| 27 | 1107 | *Staphylococcus xylosus* | Negative |
| 28 | 1111 | *Staphylococcus capitis* | Negative |
| 29 | 1112 | *Staphylococcus xylosus* | Negative |
| 30 | 1113 | *Staphylococcus sciuri* | Negative |
| 31 | 1156 | *Listeria innocua* | Negative |
| 32 | 1164 | *Listeria ivanovii* | Negative |
| 33 | 1172 | *Listeria welshimeri* | Negative |
| 34 | 2392 | *Rhodoccus equi* | Negative |
| 35 | 2552 | *Enterococcus faecium* | Negative |
| 36 | 2553 | *Enterococcus faecium* | Negative |
| 37 | 2554 | *Enterococcus faecalis* | Negative |
| 38 | 2558 | *Citrobacter freundii* | Negative |
| 39 | 2560 | *Citrobacter koseri* | Negative |
| 40 | 2561 | *Citrobacter koseri* | Negative |
| 41 | 2624 | *Enterococcus galfinarum* | Negative |
| 42 | 2625 | *Enterococcus durans* | Negative |
| 43 | 2626 | *Enterococcus hirae* | Negative |
| 44 | 2628 | *Salmonella* Kentucky | Negative |
| 45 | 2636 | *Staphylococcus felis* | Negative |
| 46 | 2874 | *Listeria seeligeri* | Negative |
| 47 | 3244 | *Listeria innocua* | Negative |
| 48 | 3327 | *Listeria seeligeri* | Negative |
| 49 | 3329 | *Listeria seeligeri* | Negative |
| 50 | 3359 | *Listeria welshimeri* | Negative |
| 51 | 3363 | *Listeria murrayi* / *grayi* | Negative |
| 52 | 3376 | *Listeria ivanovii* | Negative |
| 53 | 3572 | *Listeria innocua* | Negative |
| 54 | 3981 | *Enterococcus faecalis* | Negative |
| 55 | 3992 | *Streptococcus mutans* | Negative |
| 56 | 3996 | *Streptococcus equi* | Negative |
| 57 | 4063 | *Carnobacterium gallinarum* | Negative |
| 58 | 4064 | *Carnobacterium divergens* | Negative |
| 59 | 7332 | *Lactobacillus curvatus* | Negative |
| 60 | 7344 | *Lactobacillus acidophilus* | Negative |
| 61 | 9174 | *Microccus luteus* | Negative |

### EXAMPLE 4

| **Food Enrichment Testing** | |
|---|---|
| **Food Type** | ***Listeria monocytogenes* isolate** |
| Spinach | *L. monocytogenes* DD1307 or DD6610 |
| Smoked salmon | *L. monocytogenes* DD1302 or DD6339 |
| Ready to eat Turkey | *L. monocytogenes* DD5849 or DD6610 |
| Cheese | *L. monocytogenes* DD1302 or DD1149 |

Sensitivity and specificity of the assays were also evaluated with Listeria-spiked enrichments of different food matrices. Briefly, 25 g portions of Listeria-free food matrices were enriched in 225 mL of 24 LEB (Listeria Enrichment Broth) with antibiotic supplements. Fresh grown BHI cultures of the listed *Listeria monocytogenes* isolates were serially diluted and spiked into the respective food enrichments to achieve concentrations of 1x10⁸, 1x10⁷, 1x10⁶, 1x10⁵, 1x10⁴ and 1x10³ cfu/ml. Lysates of the spiked enrichments were prepared using the standard BAX® lysis protocol and the assay run in lyophilized tablet form. The assay showed a sensitivity of about 10⁴ cfu/mL for each *Listeria monocytogenes* enrichment lysate. No cross-reaction to the background microflora was found, as all blank food enrichment lysates tested negative by the assay.

## Claims

1. A method for detecting the presence of *Listeria monocytogenes* in a sample, said sample comprising nucleic acids, said method comprising:
(a) providing a reaction mixture comprising:
(i) suitable primer pairs for amplification of at least a portion of the putative inosose isomerase protein (IoII) genes, said primer pairs selected from the group consisting of SEQ ID NO:2 and SEQ ID NO:7, SEQ ID NO:3 and SEQ ID NO:5, SEQ ID NO:4 and SEQ ID NO:5, SEQ ID NO:5 and SEQ ID NO:6, SEQ ID NO:5 and SEQ ID NO:8, and SEQ ID NO:9 and SEQ ID NO:10, and
(ii) at least one nucleic acid probe selected from the group consisting of SEQ ID NOs:11, 12, 13, 14, 15, and 16;
(b) performing PCR amplification of said nucleic acids of said sample using the reaction mixture of step (a); and
(c) detecting the amplification of step (b), whereby a positive detection of amplification indicates the presence of *Listeria monocytogenes* in the sample.

2. The method of claim 1, wherein said at least one nucleic acid probe comprises a detectable label.

3. The method of claim 1, wherein the sample comprises a food sample or an environmental sample.

4. A kit for detection of Listeria monocytogenes serotypes in a sample, comprising:
(i) a set of primer pairs selected from the group consisting of:
(a) SEQ ID NO:2 and SEQ ID NO:7,
(b) SEQ ID NO:3 and SEQ ID NO:5,
(c) SEQ ID NO:4 and SEQ ID NO:5,
(d) SEQ ID NO:5 and SEQ ID NO:6,
(e) SEQ ID NO:5 and SEQ ID NO:8, and
(f) SEQ ID NO:9 and SEQ ID NO:10,
and
the probe of SEQ ID NO:11, SEQ ID NO: 12, SEQ ID NO:13, SEQ ID NO: 14, SEQ ID NO:15, or SEQ ID NO:16 or
(ii) the probe-primer pair of SEQ ID NO:2 and SEQ ID NO:11, SEQ ID NO:3 and SEQ ID NO:11, SEQ ID NO:4 and SEQ ID NO:11, SEQ ID NO:6 and SEQ ID NO:11, SEQ ID NO:8 and SEQ ID NO:11, SEQ ID NO:5 and SEQ ID NO:12, SEQ ID NO:5 and SEQ ID NO:13, SEQ ID NO:10 and SEQ ID NO:14, SEQ ID NO:5 and SEQ ID NO:15, or SEQ ID NO:5 and SEQ ID NO:16.

5. The kit for detection of *Listeria monocytogenes* serotypes in a sample of claim 4, wherein the kit further comprises at least one DNA polymerase.

6. The kit of claim 5 wherein the at least one DNA polymerase is an at least one thermostable DNA polymerase.

## Patentansprüche

1. Verfahren zum Nachweis des Vorkommens von *Listeria monocytogenes* in einer Probe, wobei die Probe Nukleinsäuren umfasst, wobei das Verfahren umfasst:
(a) Bereitstellen einer Reaktionsmischung, umfassend:
(i) geeignete Primerpaare zur Amplifikation von mindestens einem Teil der mutmaßlichen Inosose-Isomerase-Protein-(LoII-)Gene, wobei die Primerpaare ausgewählt sind aus der Gruppe, bestehend aus SEQ ID Nr.:2 und SEQ ID Nr.:7, SEQ ID Nr.:3 und SEQ ID Nr.:5, SEQ ID Nr.:4 und SEQ ID Nr.:5, SEQ ID Nr.:5 und SEQ ID Nr.: 6, SEQ ID Nr.: 5 und SEQ ID Nr.: 8 und SEQ ID Nr.: 9 und SEQ ID Nr.: 10 und
(ii) mindestens eine Nukleinsäuresonde, ausgewählt aus der Gruppe, bestehend aus SEQ ID Nr.: 11, 12, 13, 14, 15 und 16;
(b) Durchführen einer PCR-Amplifikation der Nukleinsäuren der Probe unter Verwendung der Reaktionsmischung aus Schritt (a); und
(c) Nachweisen der Amplifikation aus Schritt (b), wodurch ein positiver Nachweis der Amplifikation das Vorkommen von *Listeria monocytogenes* in der Probe anzeigt.

2. Verfahren nach Anspruch 1, wobei die mindestens eine Nukleinsäuresonde eine nachweisbare Markierung umfasst.

3. Verfahren nach Anspruch 1, wobei die Probe eine Lebensmittelprobe oder eine Umweltprobe umfasst.

4. Kit zum Nachweis von Listeria monocytogenes-Serotypen in einer Probe, umfassend:
(i) einen Satz von Primerpaaren, ausgewählt aus der Gruppe bestehend aus:
(a) SEQ ID Nr.: 2 und SEQ ID Nr.: 7,
(b) SEQ ID Nr.: 3 und SEQ ID Nr.: 5,
(c) SEQ ID Nr.: 4 und SEQ ID Nr.: 5,
(d) SEQ ID Nr.: 5 und SEQ ID Nr.: 6,
(e) SEQ ID Nr.: 5 und SEQ ID Nr.: 8 und
(f) SEQ ID Nr.: 9 und SEQ ID Nr.: 10,
und
die Sonde von SEQ ID Nr.: 11, und SEQ ID Nr.: 12, SEQ ID Nr.: 13, SEQ ID Nr.: 14, SEQ ID Nr.: 15 oder SEQ ID Nr.: 16 oder
(ii) das Sonden-Primerpaar von SEQ ID Nr.: 2 und SEQ ID Nr.: 11, SEQ ID Nr.: 3 und SEQ ID Nr.: 11, SEQ ID Nr.: 4 und SEQ ID Nr.: 11, SEQ ID Nr.: 6 und SEQ ID Nr.: 11, SEQ ID Nr.: 8 und SEQ ID Nr.: 11, SEQ ID Nr.: 5 und SEQ ID Nr.: 12, SEQ ID Nr.: 5 und SEQ ID Nr.: 13, SEQ ID Nr.: 10 und SEQ ID Nr.: 14, SEQ ID Nr.: 5 und SEQ ID Nr.: 15 oder SEQ ID Nr.: 5 und SEQ ID Nr.: 16.

5. Kit zum Nachweis von *Listeria monocytogenes-Serotypen* in einer Probe nach Anspruch 4, wobei das Kit weiter mindestens eine DNA-Polymerase umfasst.

6. Kit nach Anspruch 5, wobei die mindestens eine DNA-Polymerase mindestens eine wärmestabile DNA-Polymerase ist.

## Revendications

1. Procédé pour détecter la présence de *Listeria monocytogenes* dans un échantillon, ledit échantillon comprenant des acides nucléiques, ledit procédé comprenant:
(a) la fourniture d'un mélange réactionnel comprenant :
(i) des paires d'amorces appropriées pour l'amplification d'au moins une partie des gènes putatifs d'une protéine inosose isomérase (IoII), lesdites paires d'amorces étant sélectionnés dans le groupe constitué de SEQ ID NO : 2 et SEQ ID NO : 7, SEQ ID NO:3 et SEQ ID NO:5, SEQ ID NO:4 et SEQ ID NO:5, SEQ ID NO : 5 et SEQ ID NO: 6, SEQ ID NO: 5 et SEQ ID NO: 8, et SEQ ID NO: 9 et SEQ ID NO : 10, et
(ii) au moins une sonde d'acide nucléique sélectionnée dans le groupe constitué de SEQ ID NO : 11, 12, 13, 14, 15, et 16;
(b) la mise en oeuvre d'une amplification par PCR desdits acides nucléiques dudit échantillon en utilisant le mélange réactionnel de l'étape (a) ; et
(c) la détection de l'amplification de l'étape (b), selon laquelle une détection positive d'amplification indique la présence de *Listeria monocytogenes* dans l'échantillon.

2. Procédé selon la revendication 1, dans lequel ladite au moins une sonde d'acide nucléique comprend un marqueur détectable.

3. Procédé selon la revendication 1, dans lequel l'échantillon comprend un échantillon alimentaire ou un échantillon environnemental.

4. Kit pour la détection de sérotypes de Listeria monocytogenes dans un échantillon, comprenant :
(i) un jeu de paires d'amorces sélectionné dans le groupe constitué de :
(a) SEQ ID NO : 2 et SEQ ID NO : 7,
(b) SEQ ID NO : 3 et SEQ ID NO : 5,
(c) SEQ ID NO: 4 et SEQ ID NO : 5,
(d) SEQ ID NO : 5 et SEQ ID NO : 6,
(e) SEQ ID NO : 5 et SEQ ID NO : 8, et
(f) SEQ ID NO : 9 et SEQ ID NO : 10,
et
la sonde de SEQ ID NO : 11, SEQ ID NO : 12, SEQ ID NO : 13, SEQ ID NO : 14, SEQ ID NO : 15, ou SEQ ID NO : 16 ou
(ii) la paire de sonde-amorce de SEQ ID NO : 2 et SEQ ID NO : 11, SEQ ID NO : 3 et SEQ ID NO : 11, SEQ ID NO : 4 et SEQ ID NO : 11, SEQ ID NO : 6 et SEQ ID NO : 11, SEQ ID NO : 8 et SEQ ID NO : 11, SEQ ID NO : 5 et SEQ ID NO : 12, SEQ ID NO : 5 et SEQ ID NO : 13, SEQ ID NO : 10 et SEQ ID NO : 14, SEQ ID NO : 5 et SEQ ID NO : 15, ou SEQ ID NO : 5 et SEQ ID NO : 16.

5. Kit pour la détection de sérotypes de *Listeria monocytogenes* dans un échantillon selon la revendication 4, dans lequel le kit comprend en outre au moins une ADN polymérase.

6. Kit selon la revendication 5 dans lequel l'au moins une ADN polymérase est au moins une ADN polymérase thermostable.
